# EUROPEAN PATENT SPECIFICATION

(11) **EP 1 698 369 B1**
(45) Date of publication and mention of the grant of the patent: **09.12.2009**
(21) Application number: 04820289.9
(22) Date of filing: 10.12.2004
(51) Int. Cl.: A61M 25/088

(54) **CATHETER ASSEMBLY**
KATHETERANORDNUNG
ENSEMBLE DE CATHETER

(30) Priority: 15.12.2003 JP 2003417064
(43) Date of publication of application: 06.09.2006
(73) Proprietor: TERUMO KABUSHIKI KAISHA, Tokyo 151-0072 (JP)
(72) Inventor: ITOU, Takenari c/o Terumo Kabushiki Kaisha, Fujinomiya-shi, Shizuoka 4180015 (JP); FUKUOKA, Tetsuya c/o Terumo Kabushiki Kaisha, Fujinomiya-shi, Shizuoka 4180015 (JP)
(74) Representative: Casalonga, Axel
(86) International application number: PCT/JP2004/018459
(87) International publication number: WO 2005/056100

(56) References cited:
- EP-A- 0 277 366
- EP-A- 0 995 459
- JP-A- 11 500 939
- JP-A- 63 238 876
- JP-A- 2005 000 553
- JP-B2- 7 041 078

## Description

### Technical Field

The present invention relates to a catheter assembly to be used for introduction into the heart or the peripheral tissues, especially the left and right coronary arteries (coronary artery for short hereinafter). More particularly, the present invention relates to a guiding catheter to introduce a procedure catheter (e.g. a dilatation catheter for PTCA, a catheter for stent delivery or the like) to a target position of a blood vessel or also relates to a catheter to inject an angiographic medium into a target position of a blood vessel.

### Background Art

It is common practice to treat the stenosis in a blood vessel by means of a dilatation catheter (balloon catheter) or other procedure catheter. The former is designed such that the balloon inflates after insertion into a blood vessel to expand the stenotic site and improve the bloodstream beyond it. The latter is so designed as to deliver a stent (in its collapsed state) to a stenotic site and place it there after expansion. Such a catheter is inserted to and placed at the target site (stenotic site) by the procedure called PTCA (percutaneous transluminal coronary angioplasty).

The procedure catheters of these types are generally so small in diameter. Therefore the transmitting force to advance (or push) the catheter or torque to rotate the catheter by manipulation at its proximal side is presented difficulties by buckling and/or bending of the catheter.

Therefore the in PTCA procedure, a guiding catheter is usually introduced to the ostium of the coronary artery (for example, left coronary artery) where there exists the stenotic site (target site) prior to insertion of a procedure catheter. Then, the distal end of the guiding catheter is inserted into (and engaged with) the ostium of the coronary artery so that it is fixed there. This fixing step is called engaging.

After the guiding catheter has been engaged, a guide wire and a procedure catheter are inserted into the guiding catheter. The procedure catheter is allowed to protrude from the distal end of the guiding catheter for insertion into the coronary artery in which there exists the stenotic site.

The guiding catheter mentioned above needs an entrance for its introduction into a blood vessel. Therefore, it should have as small an outside diameter as possible for the minimum patient load. It should also have as large an inside diameter as possible for convenient handling of the procedure catheters to be inserted thereinto.

However, with an excessively thin wall to meet these requirements, the guiding catheter is too weak to withstand kinking when it is inserted in a meandering portion of the blood vessel. Moreover, the guiding catheter should have adequate rigidity for good pushability and torque transmission to the distal end.

In order to address the above-mentioned problems, new technologies have been developed. One of them is to embed braided wires in the wall of the guiding catheter (see JP SHO 58-149 766, and the other is to use braided wires in a flat form (see JP-HEI-10-127773) . The recent trend is toward inserting the catheter through the artery of the arm for the better patient's QOL (Quality Of Life) after operation. Thus there is an increasing demand for a guiding catheter with a thinner wall than before.

Furthermore, a guiding catheter with an excessively thin wall has a sharp tip which might damage the blood vessel at the time of insertion.

Thus it is difficult to obtain an ideal guide catheter that meets the contradictory requirements mentioned above.

EP -A 0 277 366 discloses a catheter assembly comprising an outer catheter and an inner catheter that can be inserted, in to said outer catheter. The outer catheter has a flexible soft tip. The outer catheter hub may be fixed with a plastic fitting on the inner catheter tube. A part of the inner catheter protrudes from a distal end of the outer catheter. Such a construction is not entirely satisfactory.

### Disclosure of Invention

It is an object of the present invention to provide a catheter assembly which has flexibility at the distal end as well as sufficient strength, permits safe and sure insertion to the desired position by simple operation, and accommodates a variety of procedure catheters by its enlarged inside diameter.

As claimed, the present invention to achieve the above-mentioned object is directed to a catheter assembly comprising an outer catheter and an inner catheter that can be inserted into said outer catheter, said outer catheter being comprised an outer catheter body comprising at least an inner layer, an outer layer, and a reinforcing layer interposed between them, a flexible soft tip attached to a distal end of said outer catheter body, and an outer catheter hub attached to a proximal end of said outer catheter body, said inner catheter being comprised a hard proximal part, a distal part softer than said hard proximal part, and an inner catheter hub formed at a proximal end of the proximal part, said outer catheter hub and said inner catheter hub are adapted to be fixed each other so that said two catheters do not rotate and move relative to each other, when said outer catheter hub and said inner catheter hub engage each other at least a part of said inner catheter protrudes from a distal end of said outer catheter, According to the invention, the distancebetween the distal end of said outer catheter and a distal end of said inner catheter is no more than 10 mm.

The catheter assembly has no possibility of damaging the vessel wall because it is inserted to the desired site in the blood vessel in such a way that the outer catheter and the inner catheter behave as one body and it permits the distal end (the second soft tip) of the inner catheter to slightly protrude from the distal open end of the outer catheter while ensuring good operability (pushability and torque transmission) and good kink resistance. It is to be noted that, it can be used in the same way as an ordinary catheter because of the very short distance between the distal end of the outer catheter and the distal end of the inner catheter. When the inner catheter is withdrawn after arrival at the desired site, the distal end of the outer catheter approximately coincides with the distal end of the catheter assembly. In addition, the distal end of the inner catheter is so flexible that the bent shape of the outer catheter remains unchanged. Thus the catheter assembly can be inserted to the desired site rapidly, surely, and safely.

After the catheter hubs disengaged and the inner catheter is removed, the outer catheter leaves a large space inside diameter suitable for the procedure catheter. Thus the outer catheter can have a very thin wall thickness which has never been realized before.

### Brief Description of Drawings

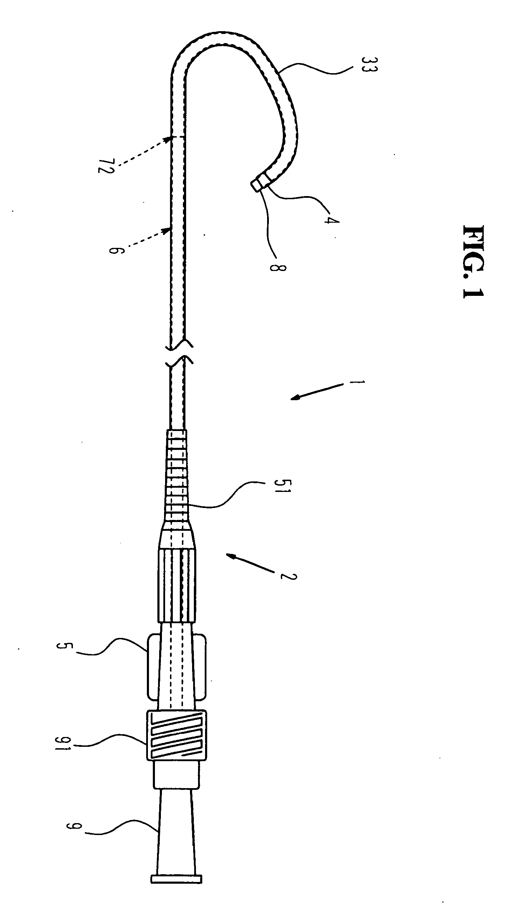

[Fig. 1] Fig. 1 is a plan view showing the entire configuration of the catheter assembly.
[Fig. 2]

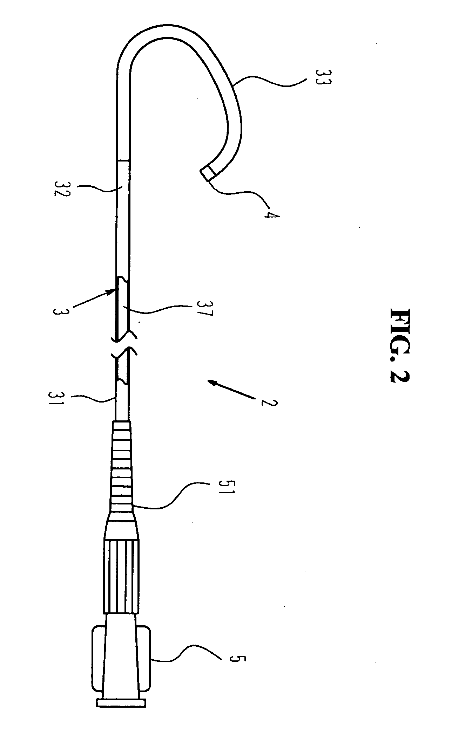

   Fig. 2 is a plan view showing the entire configuration of the outer catheter of the catheter assembly.
[Fig. 3]

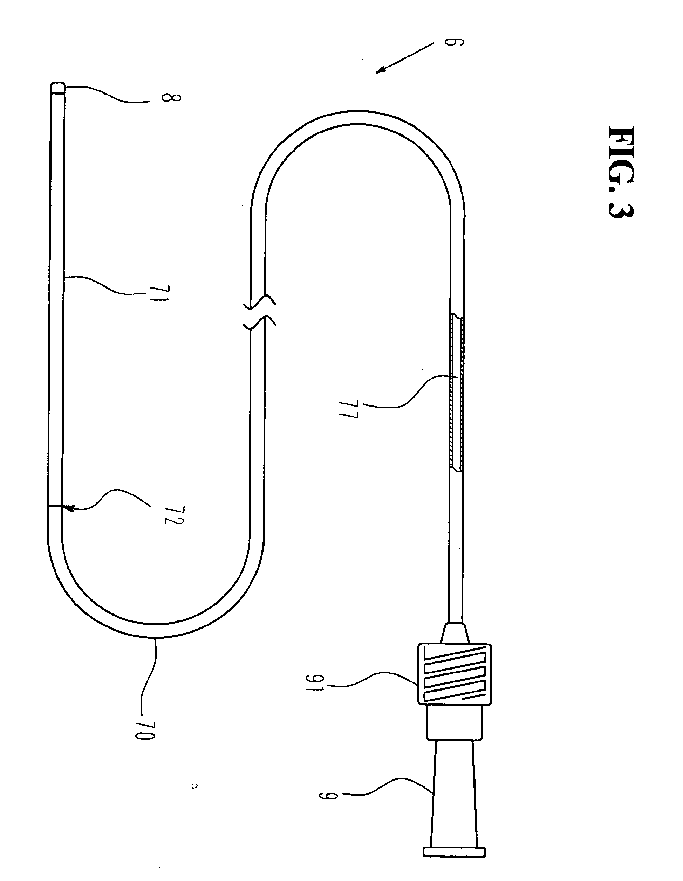

   Fig. 3 is a plan view showing the entire configuration of the inner catheter of the catheter assembly.
[Fig. 4]

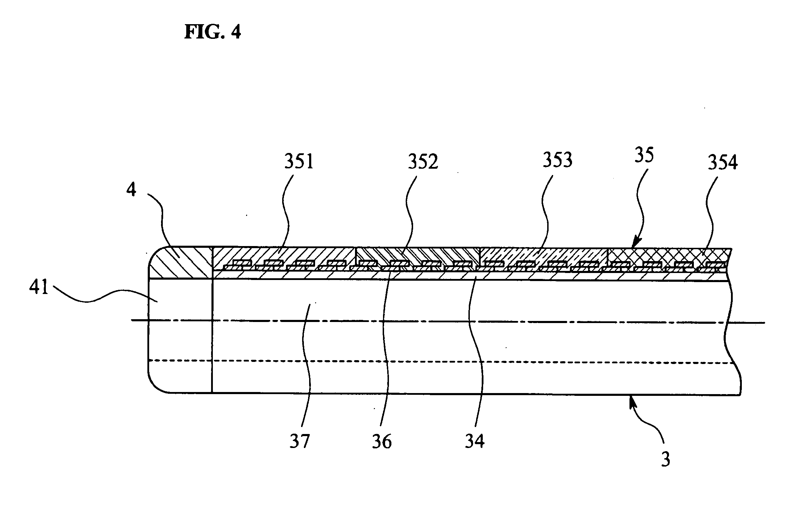

   Fig. 4 is a partial sectional view showing the structure of the distal end of the outer catheter body.
[Fig. 5]

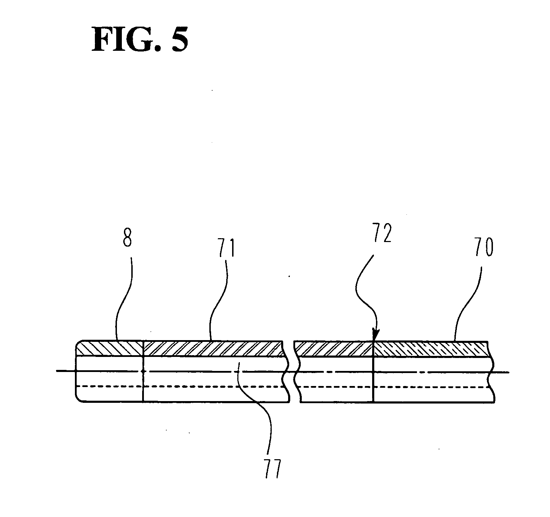

   Fig. 5 is a partial sectional view showing the structure of the distal end of the inner catheter body.
[Fig. 6]

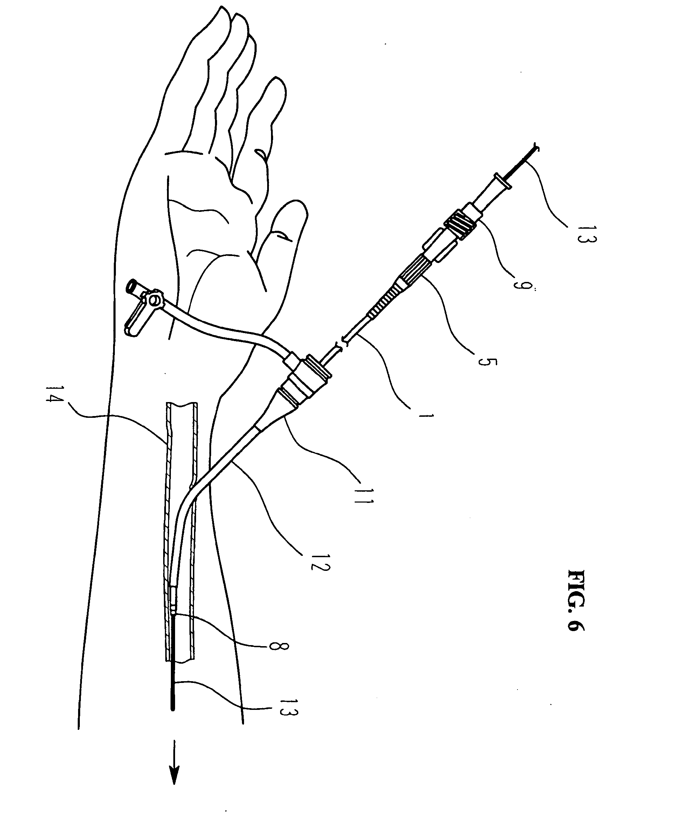

   Fig. 6 is a diagram illustrating how to introduce into the blood vessel the catheter assembly.
[Fig. 7]

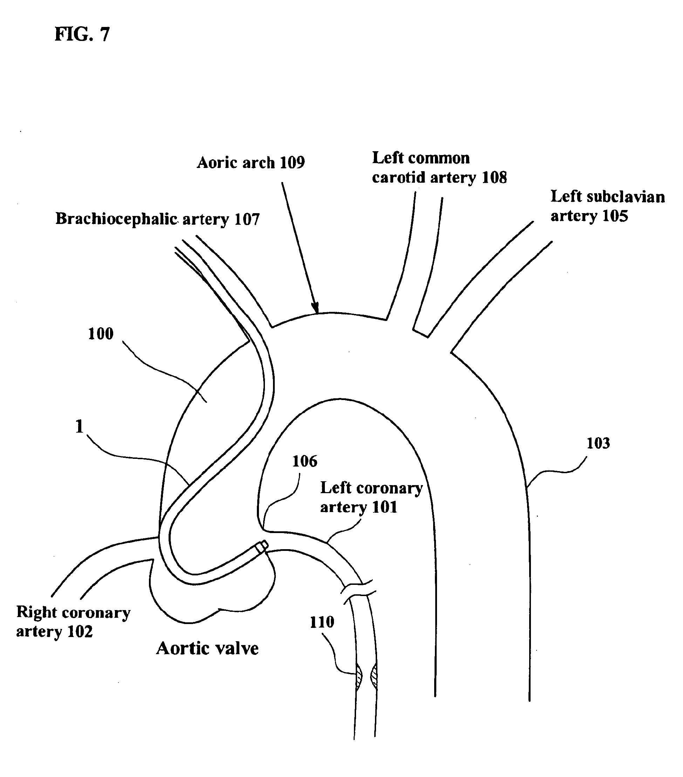

   Fig. 7 is a schematic diagram illustrating how to insert and place in the left coronary artery the catheter assembly.
[Fig. 8]

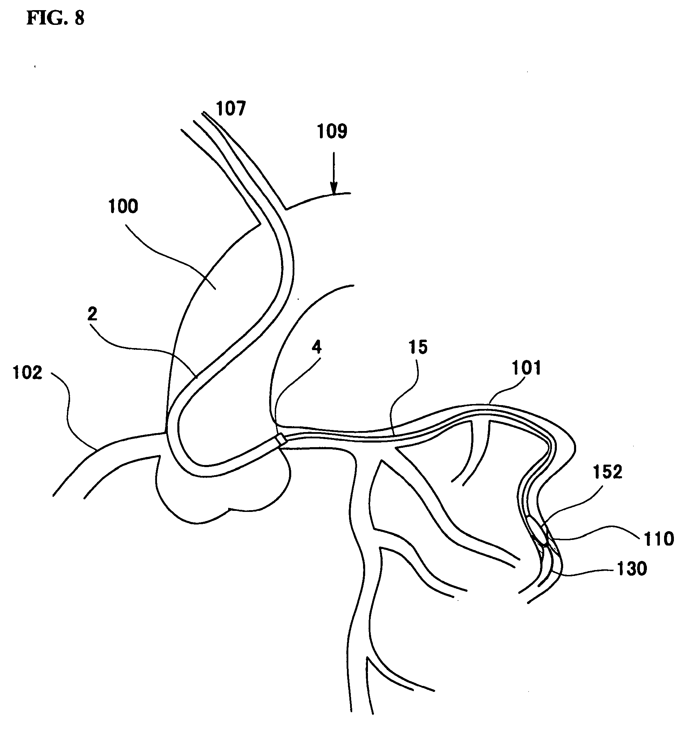

   Fig. 8 is a schematic diagram illustrating how to insert and place in the left coronary artery the catheter assembly.

### Description of Reference Characters

- 1: Catheter assembly
- 2: Outer catheter
- 3: Outer catheter body
- 31: Proximal part
- 32: Intermediate part
- 33: Distal part
- 34: Inner layer
- 35: Outer layer
- 351: First region
- 352: Second region
- 353: Third region
- 354: Fourth region
- 36: Reinforcing layer
- 37: Lumen
- 4: Soft tip
- 41: Distal opening
- 5: Hub
- 6: Inner catheter
- 70: Inner catheter body
- 71: Distal end of inner catheter
- 72: Boundary
- 77: Lumen
- 8: Soft tip
- 9: Hub
- 11: Catheter introducer
- 12: Sheath
- 13: Guide wire
- 14: Artery
- 15: Balloon catheter
- 151: Distal end
- 152: Balloon
- 100: Ascending aorta
- 101: Left coronary artery
- 102: Right coronary artery
- 103: Abdominal aorta
- 104: Left inner wall
- 105: Left subclavian artery
- 106: Left coronary artery ostium
- 107: Brachiocephalic trunk
- 108: Left common carotid artery
- 109: Aortic arch
- 110: Stenotic site

### Best Mode for Carrying out the Invention

In what follows, the catheter assembly according to the present invention will be described in more detail with reference to the preferred embodiments illustrated in the accompanying drawings.

Incidentally, this specification uses the term "proximal" and "distal" to note respectively the right side and the left side in Figs. 1 to 5. Moreover, this specification uses the term "near position" and "far position" to note respectively the side close to the proximal end and the side far from the proximal end.

The catheter assembly 1 shown in Fig. 1 is designed to be used as a guiding catheter to guide a procedure catheter to a target site or as an angiographic catheter to inject a contrast medium into a target site of a blood vessel. The procedure catheter such as a dilatation catheter (i.e. balloon catheter) or a catheter for stent transportation to a target site of a blood vessel is used to dilate a stenotic site in the coronary artery by the balloon or the stent.

The catheter assembly 1 is composed mainly of an outer catheter 2 and an inner catheter 6.

The outer catheter 2 is composed of an outer catheter body 3, a first soft tip 4, which is flexible and is attached to the distal end of the outer catheter body 3, and a hub (outer catheter hub) 5 at the proximal end of the outer catheter body 3.

The outer catheter body 3 is a flexible tube which has a lumen 37 formed approximately the center thereof and over the entire length thereof. The lumen 37 opens at the distal end of the first soft tip 4.

As shown in Fig. 4, the tube constituting the outer catheter body 3 is composed of three laminated layers, which are an inner layer 34, an outer layer 35, and a reinforcing layer 36 interposed between them.

The outer layer 35 is composed of the following four regions. A first region 351. A second region 352 which is closer to the proximal end than the first region 351. A third region 353 which is closer to the proximal end than the second region 352. A fourth region 354 which is closer to the proximal end than the third region 353. The third region 353 is more flexible than the fourth region 354. The second region 352 is more flexible than the third region 353. The first region 351 is more flexible than the second region 352. Because of this structure, the outer catheter body 3 gradually increases in flexibility in going toward the distal end, so that the catheter assembly can be inserted into the blood vessel safely with adequate pushability and torque transmission to the distal end.

The first to fourth regions 351, 352, 353, and 354 may be formed from any of various thermoplastic elastomers such as styrene, polyolefin, polyurethane, polyester, polyamide, polybutadiene, trans-polyisoprene, fluororubber, and chlorinated polyethylene. They may be used alone or in combination with one another (in the form of polymer alloy, polymer blend, or laminate).

The inner layer 34 may be formed from any material which is not specifically restricted. However, it is desirable to use a low-friction material for at least that part of the inner layer 34 that comes into contact with the inner catheter 6 when the inner catheter 6 is inserted into the lumen 37 (in the outer catheter body 3). The resulting inner layer 34 permits the inner catheter 6 to be moved (inserted) in the lengthwise direction with a less sliding resistance from the outer catheter body 3. Moreover, it also permits the procedure catheter to move and rotate with a less sliding resistance, thereby contributing to operability.

Examples of the low-friction material include a variety of plastic materials, such as polyamide, polyether polyamide, polyester polyamide, polyester (e.g., polyethylene terephthalate, polybutylene terephthalate, and polyethylene naphthalate), polyurethane, flexible polyvinyl chloride, ABS resin, AS resin, and fluoroplastics such as polytetrafluoroethylene.

The reinforcing layer 36 comprises a reinforcing material to reinforce the outer catheter body 3. The reinforcing material may be one which is formed from filament such as in helical shape or braid. The filamentous reinforcing material may be metal or hard resin or the like. Its typical example is a flattened thin wire of stainless steel which is helically wound or braided so that the outer catheter body 3 has a small wall thickness in the radial direction.

Owing to the reinforcing layer 36, the outer catheter body 3 has a comparatively large inside diameter (or the diameter of the lumen 37) and sufficient rigidity and strength without the necessity of increasing the wall thickness of the outer catheter 3. As the result, the outer catheter body 3 permits insertion of the inner catheter body 70 with a comparatively larger outside diameter, and the outer catheter 2 excels in pushability and torque transmission and resists kinking and crushing.

Incidentally, the number and material of layers constituting the outer catheter body 3 may vary along its length, and the reinforcing material may be present or absent at different parts of the outer catheter body 3. For example, the distal part 33 of the outer catheter body 3 may be composed of a softer material or a less number of layers or may be free of the reinforcing material so that it is made more flexible.

The outer layer 35 should preferably be formed from a material incorporated with an X-ray contrast medium (radiopaque material) because the catheter assembly 1 is inserted into the body while its position is being confirmed with the help of radioscopy. Examples of the radiopaque material include barium sulfate, bismuth oxide, and tungsten. An amount of 30 to 80 wt% is adequate for the radiopaque material in the constituent material of the outer layer 35.

The radiopaque material may be included in the entire length of the outer catheter body 3 or may be included only in a part of the outer catheter body 3, for example, the distal part 33 or the first soft tip 4.

The inner layer 34 may be formed from the low-friction material entirely instead of partly for the part in contact with the inner catheter 6 as mentioned above.

The outer catheter body 3 has the proximal part 31, the intermediate part 32, and the distal part (curved portion) 33 having a curved form as desired. The proximal part 31 and the intermediate part 32 extend almost linearly from the proximal end in the longitudinal direction. The distal part 33 extends further from the intermediate part 32. The distal part 33 has a curved form suited for its insertion into the left or right coronary artery. The curved distal part 33, therefore, is easily engaged with and remains engaged with the ostium of the coronary artery.

The distal part 33 should preferably have at least the first region 351 out of the first to fourth regions 351, 352, 353, and 354 mentioned above.

To the curved distal part 33 is connected with the first soft tip 4, which is made of a flexible material and has a round shape. The first soft tip 4 ensures smooth and safe threading through a curved, bent, or branched vessel.

The first soft tip 4 may be formed from a rubber, such as natural rubber, isoprene rubber, butadiene rubber, chloroprene rubber, silicone rubber, fluororubber, and styrene-butadiene rubber, or a thermoplastic elastomer, such as styrene, polyolefin, polyurethane, polyester, polyamide, polybutadiene, trans-polyisoprene, fluororubber, and chlorinated polyethylene.

The constituent material for the first soft tip 4 may be incorporated with a radiopaque material (X-ray contrast medium) mentioned above.

The first soft tip 4 is not specifically restricted in length; however, it should preferably be about 0.5 to 3 mm long, more preferably about 1 to 2 mm long.

To the proximal end of the outer catheter body 3 is attached (fixed) the hub 5, which has a bore communicating with the lumen 37. The bore has about the same inside diameter as the lumen 37, so that there are no steps between the lumen 37 and the inside of the proximal end.

The hub 5 permits introduction and withdrawal of a long item or filamentous body, such as guide wire, catheter (e.g. balloon catheter for PTCA or stent transporting catheter), endoscope, ultrasonic probe, and temperature sensor, and also permits injection of various fluids (such as contrast medium, drug solution, and normal saline). In addition, the hub 5 may be connected to any other instrument to measure, for example, blood pressure.

The connecting part for the outer catheter body 3 and the hub 5 is covered with an elastic material (kink-resisting protector 51), so that it is protected from kinking around the connecting part.

The inner catheter 6 is comprised of the following four components: the inner catheter body 70, which is closed to the proximal side; the inner catheter distal part 71 which extends from the inner catheter body 70; the second soft tip 8 which is flexible and extends further from the inner catheter distal part 71; and the hub 9 which is attached to the proximal end of the inner catheter body 70.

The inner catheter body 70 is a flexible tube, which has the lumen 77 formed at its center over its entire length. The lumen 77 opens at the distal end of the second soft tip 8.

The tube constituting the inner catheter 6 is comprised of a comparatively rigid single-layer resin tube which constitutes the inner catheter body 70, a comparatively flexible single-layer resin tube which constitutes the inner catheter distal part 71, and a second soft tip 8 which is formed from a softer resin than that constituting the inner catheter distal part 71.

The inner catheter distal part 71 is more flexible than the inner catheter body 70, and the second soft tip 8 is more flexible than the inner catheter distal part 71. The inner catheter 6 constructed in this manner is rigid enough (at the proximal side) to firmly support the outer catheter 2 for improved kink resistance, pushability, and torque transmission. The flexible distal part does not greatly deform the curved part 33 of the outer catheter 2. Thus, the boundary between the inner catheter distal part 71 and the inner catheter body 70 is positioned closer to the proximal side than the base of the curved part 33 when the inner and outer catheters 6 and 2 are assembled. Moreover, the second soft tip 8 ensures safety for the vessel wall.

The inner catheter body 70 and the inner catheter distal part 71 may be formed from any of various thermoplastic elastomers such as styrene, polyolefin, polyurethane, polyester, polyamide, polybutadiene, trans-polyisoprene, fluororubber, and chlorinated polyethylene. They may be used alone or in combination with one another (in the form of polymer alloy or polymer blend).

The inner catheter body 70 and the inner catheter distal part 71 may be formed from the same kind of material or different materials.

The resin used for the inner catheter body 70 should preferably be one which has a Shore D hardness of 50 to 80D (according to ASTM-D2240). This material provides the inner catheter body 70 with adequate rigidity to prevent kinking and twisting when inserted into the blood vessel in combination with the outer catheter 2.

The resin used for the inner catheter distal part 71 should preferably be one which has a Shore D hardness of 25 to 50D (according to ASTM-D2240). This material prevents the forward curved part 33 of the outer catheter 2 from greatly deforming when the inner catheter 6 is combined with the outer catheter 2.

Because the inner catheter body 70 is inserted into the living body under radioscopy, the constituting material for the outer layer 75 should preferably be incorporated with a radiopaque material (contrast medium), such as barium sulfate, bismuth oxide, and tungsten. An amount of 30 to 80 wt% is preferred for the radiopaque material in the constituent material of the outer layer 75.

The radiopaque material may be present over the entire length of the inner catheter 6 or present partly along the length, for example, in the inner catheter distal part 71 or a part containing it, or in the second soft tip 8.

The second soft tip 8 connected to the distal end of the inner catheter 6 is made of a flexible material, and it should preferably has a round end. In addition, the second soft tip 8 may have a thicker wall than the first soft tip 4 so as to ensure safer insertion into the blood vessel (e.g. inner wall of coronary artery) when the inner catheter 6 is inserted to the coronary artery, and it also permits smooth threading through bending blood vessels.

The soft tip 8 may be formed from a rubber, such as natural rubber, isoprene rubber, butadiene rubber, chloroprene rubber, silicone rubber, fluororubber, and styrene-butadiene rubber, or a thermoplastic elastomer, such as styrene, polyolefin, polyurethane, polyester, polyamide, polybutadiene, trans-polyisoprene, fluororubber, and chlorinated polyethylene.

The constituent material for the soft tip 8 may be incorporated with a radiopaque material mentioned above.

The soft tip 8 is not specifically restricted in length; however, it should preferably be about 0.5 to 3 mm long, more preferably about 1 to 2 mm long.

To the proximal end of the inner catheter body 70 is attached (fixed) the hub (i.e. the inner catheter hub) 9, which has a bore communicating with the lumen 77. The bore has about the same inside diameter as the lumen 77, so that there are no steps between the bore and the inner surface of the proximal end of the lumen 77.

The hub 9 permits introduction and withdrawal of a long item or filamentous body, such as guide wire, catheter (for example, balloon catheter for PTCA, and stent transporting catheter), endoscope, ultrasonic probe, and temperature sensor, and also permits injection of various fluids (such as contrast medium, drug solution, and normal saline). In addition, the hub 9 may be connected to any other instrument to measure, for example, blood pressure.

In addition, the inner catheter hub 9 has a Luer-Lock connector 91 for its integral connection with the outer catheter hub 5. The Luer-Lock connector 91 has on its inner surface a screw groove which engages with the flange at the proximal end of the outer catheter hub 5. This screw groove makes the two hubs integral as they are turned relative to each other, that is, the inner catheter 6 and the outer catheter 2 are made integral. Thus, the two catheters are fixed to each other so as not to rotate and move each other. The operator only needs to manipulate one of the hubs to insert the catheter assembly 1 into the blood vessel as if he manipulates single catheter.

The inner catheter 6 is longer than the outer catheter 2 so that the distal end of the inner catheter 6 protrudes by 10 mm or less, preferably 0.5 to 5 mm, from the distal end of the outer catheter 2 when the inner catheter 6 is inserted into the outer catheter 2 and their hubs are fixed. Thus, when the two catheters are combined together, the distal end of the inner catheter 6 functions as the distal end of the catheter assembly 1. Being not so severely restricted in wall thickness as the outer catheter 2, the inner catheter 6 may have a sufficient wall thickness that ensures safety for the vessel wall. The small distance between the distal end of the outer catheter 2 and the distal end of the inner catheter 6 prevents the distal end of the outer catheter 2 from being displaced from the target site when the inner catheter 6 is withdrawn from the outer catheter 2 after the catheter assembly 1 has been placed at the target site (such as the ostium of the coronary artery) where the vessel branches from a large space into a small passage.

The outer catheter body 3 should have an outside diameter (D1 mm) and an inside diameter (d1 mm) such that the ratio of d1/D1 is from 0.89 to 0.95, preferably from 0.90 to 0.92. If this ratio is smaller than specified above, the outer catheter body 70 has a large wall thickness and a small inside diameter, thereby limiting the devices (such as procedure catheters) to be inserted into the outer catheter 2. If this ratio is larger than specified above, the outer catheter body 3 does not have a sufficient wall thickness for the back-up strength and kink resistance which are required when the procedure catheter is used.

The outside diameter of the inner catheter 6 is not specifically restricted so long as it is smaller than the inside diameter of the outer catheter 2, and the inside diameter of the inner catheter 6 is not specifically restricted so long as it is large enough for the guide wire to be inserted.

The actual dimensions are as follows. The outside diameter D1 of the outer catheter body 3 should be about 1.5 to 2.7 mm, more preferably about 1.7 to 2.4 mm. The outer catheter body 3 with an excessively large outside diameter D1 is poor in operability for insertion into and threading through the artery, and increases the patient's load.

The inside diameter d2 of the outer catheter 3 should preferably be about 1.4 to 2.4 mm, more preferably 1.5 to 2.2 mm. If the inside diameter d1 is excessively small, the inner catheter 6 is limited in the outside diameter accordingly and hence the procedure catheter to be inserted into the inner catheter 6 is also limited in the outside diameter. Thus, the selection of the procedure catheter is limited.

For example, the catheter assembly 1 according to the present invention will be used in the following way.

First, the catheter introducer 11 is penetrated into the arm artery (introducing site) 14, such as right brachial artery and right radial artery, according to Seldinger's method as shown in Fig. 6. Then, the catheter assembly 1 is inserted into the sheath 12 of the catheter introducer 11. The guide wire 13 is previously inserted into the lumen 77 of the inner catheter 6. With the guide wire 13 preceding, the distal end of the outer catheter 3 is inserted into the artery 14 through the forward opening of the sheath 12.

The distal end of the catheter assembly 1 is gradually advanced in the direction of the arrow shown in Fig. 6, with the guide wire 13 preceding, to the desired position for insertion and placement (such as the ostium of the right or left coronary artery in the aortic arch 109). This operation is accomplished by pushing or pulling the guide wire and advancing or retracting or rotating the catheter assembly 1 for smooth passage through the bending part of the vessel and for correct selection of the branch of the vessel.

The inner catheter 6 of the catheter assembly 1 is inserted into the left coronary artery ostium 106 by the operation (technique) explained below with reference to Fig. 7. Incidentally, the operation is accomplished by confirming the position and the posture of the catheter assembly 1 with the help of radioscopy.

The catheter assembly 1 is inserted into the catheter introducer 11, with the catheter introducer 11 stuck into the right brachial artery and the guide wire 13 inserted into the lumen 77 of the catheter assembly 1 (the inner catheter 6). The distal part 33 of the outer catheter 2 is nearly straight when the guide wire 13 protrudes from the distal end of the outer catheter body 3 by the stiffness of the guide wire 13.

After the catheter assembly 1 has been inserting into the right brachial artery through the catheter introducer 11, the distal end of the catheter assembly 1 is advanced from the brachiocephalic artery 107 to the ascending aorta 100, with the guide wire 13 preceding. As soon as the soft tip 8 reaches the position about 10 cm above the ostium 106 of the left coronary artery 101, the catheter assembly 1 is stopped and the guide wire 13 is withdrawn, so that the distal part 33 of the outer catheter 2 is restored to its original curved shape (i.e. natural shape).

The catheter assembly 1 is slowly advanced while confirming the position of its distal end (i.e. the position of the soft tip 8). The distal part 33 of the outer catheter 2 moves downward, while remaining in contact with the left inner wall 104 of the ascending aorta 100, and enters the left coronary artery ostium 106. At this time, the distal part 33 takes on an easy-to-engage shape.

Incidentally, in the case when the distal part 33 of the outer catheter 2 points in the opposite direction of the left coronary artery ostium 106, the outer catheter 2 is slightly turned in the counterclockwise direction so that the distal part 33 points to the left coronary artery ostium 106, and then the outer catheter 2 is slowly advanced. Thus, the distal part 33 easily enters the left coronary artery ostium 106 and is engaged in that state.

As the result of the foregoing procedure, the distal end (i.e. soft tip 8) of the catheter assembly 1 is inserted (about 5 to 15 mm) into the left coronary artery ostium 106. After engagement, the Luer-Lock 91 of the inner catheter hub 9 is released and the inner catheter 6 is withdrawn, with the outer catheter 2 remaining in the vessel.

To the outer catheter hub 5 is connected with a connector of infusion apparatus (not shown) for contrast-medium injection, and a contrast medium is injected. The injected contrast medium passes through the hub 5 and the lumen 37 and flows into the left coronary artery 101 from the forward opening 41 of the soft tip 4. In this way the stenotic site (affected part) 110 in the left coronary artery is made visible and its position is identified.

Next, the connector of infusion apparatus for contrast medium is removed from the hub 5. A procedure catheter, such as balloon catheter 15 for PTCA, is inserted, together with a new treatment guide wire 130, into the lumen 37 of the outer catheter 2, and treatment for the stenotic site 110 as a target site is carried out.

To be concrete, the balloon portion of the balloon catheter 15 is advanced to the stenotic site 110, with the guide wire 130 preceding, and the balloon 152 is dilated to cure the stenotic site 110.

The foregoing technique may be applied to inserting the catheter assembly of the present invention into the heart through the left common carotid artery 108 or the left subclavian artery 105 or through the femoral artery and the abdominal aorta 103, although this technique is different from the illustrated one.

It is also possible to inject a contrast medium through the lumen 77 of the inner catheter 6 while the catheter assembly 1 is in such a state that the inner catheter 6 is inserted into the outer catheter 2.

Incidentally, the above-mentioned technique is applicable to the procedure in which the balloon catheter 15 for PTCA is used as the procedure catheter. However, the technique is also applicable to a stent transporting catheter to transport a stent and place it at the stenotic site.

The same technique as mentioned above may also be used to expand and treat the stenotic site in the right coronary artery 102.

The catheter assembly 1 of the present invention is not restricted in its use to that mentioned above. For example, it may be applied to the guiding catheter to introduce an atherectomy catheter or ultrasonic catheter, the catheter to administer a drug solution (such as thrombolytic agent), and the catheter for angiography. It is needless to say that the site of the living body to which the catheter assembly is inserted is not limited to the coronary artery.

The specific example of the present invention will be described below in more detail.

The catheter assembly (shown in Figs. 1 to 3) was prepared which is comprised of an outer catheter and an inner catheter as specified below.

1 : Outer catheter body
Overall length : 1000 mm (excluding hub)
Outside diameter D1 : 2.06 mm
Inside diameter (lumen diameter) d1 : 1.85 mm
Length of the first region of the outer layer : 10 mm
Material of the first region of the outer layer : polyester elastomer (Shore D hardness = 44) incorporated with tungsten filler
Length of the second region of the outer layer : 20 mm
Material of the second region of the outer layer : polyester elastomer (Shore D hardness = 46)
Length of the third region of the outer layer : 30 mm
Material of the third region of the outer layer : polyester elastomer (Shore D hardness = 57) Length of the fourth region of the outer layer: 938 mm
Material of the fourth region of the outer layer : polyester elastomer (Shore D hardness = 78) Reinforcing material : braid of stainless steel wire with a flat cross section
Material of the inner layer : polytetrafluoroethylene
Soft tip : polyester elastomer incorporated with tungsten filler
Length of the soft tip : 2 mm
Shape of the distal part : Judkins left type
Length of the hub : 20 mm
2 : Inner catheter body
Overall length : 1026 mm (excluding hub)
Outside diameter D2 : 1.78 mm
Inside diameter (lumen diameter) d2 : 1.15 mm
Length of the inner catheter body (base side) : 900 mm
Material of the inner catheter body: polyester elastomer (Shore D hardness = 57) incorporated with tungsten filler
Length of the distal part of the inner catheter : 124 mm
Material of the distal part of the inner catheter : polyester elastomer (Shore D hardness = 38) incorporated with tungsten filler
Soft tip : polyurethane elastomer incorporated with tungsten filler
Length of the soft tip : 2 mm
Shape of the distal part : straight
Length of the hub : 27 mm
The above-specified catheter assembly according to the specific example has a d1/D1 ratio of 0.9 and permits the inner catheter to protrude by 3 mm from the outer catheter.

<Clinical test> A catheter introducer (with a sheath of 7 Fr = 2.34 mm in outside diameter) was percutaneously placed in the right radial artery of three patients A, B, and C according to the above-mentioned technique. The catheter assembly according to the specific example was introduced through the sheath and inserted into the blood vessel. The distal end of the inner catheter was engaged at the ostium of the left coronary artery, and then a contrast medium was injected for angiography. A clear image was obtained.

Subsequently, the inner catheter was withdrawn from the outer catheter. A balloon catheter (0.9 mm in outside diameter) for PTCA was inserted into the outer catheter, with the guide wire preceding. The balloon catheter was advanced such that its distal part (i.e. balloon portion) protruded from the forward opening of the outer catheter, and the balloon was placed before the desired position (e.g. stenotic site). Then the balloon catheter was advanced further, with the guide wire preceding, until the balloon coincided with the stenotic site. The balloon was inflated to treat the stenotic site. This technique permits the balloon to pass rapidly, safely, and smoothly without stacking (i.e. irregular passage) in the coronary artery. Moreover, the distal end of the outer catheter which had once engaged did not slip off from the ostium of the left coronary artery.

## Claims

1. A catheter assembly comprising:
an outer catheter (2) and an inner catheter (6) that can be inserted into said outer catheter;
said outer catheter being comprised an outer catheter body (3) comprising at least an inner layer (34), an outer layer (35), and a reinforcing layer (36) interposed between them, a flexible soft tip (4) attached to a distal end of said outer catheter body, and an outer catheter hub (5) attached to a proximal end of said outer catheter body;
said inner catheter (6) being comprised a hard proximal part, a distal part softer than said hard proximal part, and an inner catheter hub (9) formed at a proximal end of the proximal part, and
wherein said outer catheter hub (5) and said inner catheter hub (9) are adapted to be fixed each other so that said two catheters do not rotate and move relative to each other, when said outer catheter hub and said inner catheter hub engage each other, at least a part of said inner catheter protrudes from a distal end of said outer catheter **characterized in that** the distance between the distal end of said outer catheter and a distal end of said inner catheter is no more than 10 mm.

2. The catheter assembly as set forth in Claim 1, wherein the inner catheter comprises a second soft tip (8) with flexibility at the distal end.

3. The cacheter assembly as set forth in Claim 2, wherein the second soft tip comprises a larger wall thickness than the soft tip of the outer catheter.

4. The catheter assembly as set forth in any of Claims 1 to 3, wherein the distal part of the outer catheter body comprises a predetermined curved portion.

5. The catheter assembly as set forth in Claim 4, wherein the outer catheter hub is engaged with the inner catheter hub in such a way that the boundary between the hard proximal end and the flexible distal end of the inner catheter is located on the proximal side from the curved portion of the outer catheter body.

6. The catheter assembly as set forth in any of Claims 1 to 5, wherein the inner catheter is of single-layer stucture made of a resin containing no reinforcing material over the entire length.

7. The catheter assembly as set forth in any of Claims 1 to 6, wherein the outer catheter comprises a size such that the ratio of the outside diameter to the inside diameter is no smaller than 0.89 and no larger than 0.95.

8. The catheter assembly as set forth in any of Claims 1 to 7, wherein the outer catheter comprises a wall thickness smaller than that of the inner catheter.

## Patentansprüche

1. Katheterbaugruppe, die Folgendes umfasst:
einen Außenkatheter (2) und einen Innenkatheter (6), der in den Außenkatheter eingeführt werden kann;
wobei der Außenkather aus einem Außenkatheterkörper (3) besteht, der mindestens eine innere Schicht (34), eine äußere Schicht (35) und eine dazwischen angeordnete Verstärkungsschicht (36) umfasst sowie eine an einem distalen Ende des Außenkatheterkörpers befestigte biegsame weiche Spitze (4) und eine an einem proximalen Ende des Außenkatheterkörpers befestigte Außenkatheternabe (5);
wobei der Innenkatheter (6) aus einem harten proximalen Teil, einem distalen Teil, der weicher ist als der harte proximale Teil, und einer an einem proximalen Ende des proximalen Teils ausgebildeten Innenkatheternabe (9) besteht, und
wobei die Außenkatheternabe (5) und die Innenkatheternabe (9) aneinander befestigt werden können, so dass sich die beiden Katheter nicht zueinander drehen und bewegen, wenn die Außenkatheternabe und die Innenkatheternabe miteinander in Eingriff stehen und wenigstens ein Teil des Innenkatheters aus einem distalen Ende des Außenkatheters herausragt, **dadurch gekennzeichnet, dass** der Abstand zwischen dem distalen Ende des Außenkatheters und einem distalen Ende des Innenkatheters nicht mehr als 10 mm beträgt.

2. Katheterbaugruppe nach Anspruch 1, wobei der Innenkatheter eine zweite weiche Spitze (8) mit Biegsamkeit am distalen Ende umfasst.

3. Katheterbaugruppe nach Anspruch 2, wobei die zweite weiche Spitze eine größere Wandstärke besitzt als die weiche Spitze des Außenkatheters.

4. Katheterbaugruppe nach einem der Ansprüche 1 bis 3, wobei der distale Teil des Außenkatheterkörpers einen vorbestimmten gekrümmten Abschnitt umfasst.

5. Katheterbaugruppe nach Anspruch 4, wobei die Außenkatheternabe mit der Innenkatheternabe so in Eingriff steht, dass die Grenze zwischen dem harten proximalen Ende und dem biegsamen distalen Ende des Innenkatheters auf der von dem gekrümmten Abschnitt des Außenkatheterkörpers aus gesehen proximalen Seite liegt.

6. Katheterbaugruppe nach einem der Ansprüche 1 bis 5, wobei der Innenkatheter einen einlagigen Aufbau aus einem Harz ohne Verstärkungsmaterial über die gesamte Länge besitzt.

7. Katheterbaugruppe nach einem der Ansprüche 1 bis 6, wobei der Außenkatheter eine solche Größe besitzt, dass das Verhältnis des Außendurchmessers zum Innendurchmesser nicht kleiner ist als 0,89 und nicht größer als 0,95.

8. Katheterbaugruppe nach einem der Ansprüche 1 bis 7, wobei der Außenkatheter eine geringere Wandstärke besitzt als der Innenkatheter.

## Revendications

1. Ensemble de cathéter comprenant :
un cathéter extérieur (2) et un cathéter intérieur (6) pouvant être inséré dans ledit cathéter extérieur ;
ledit cathéter extérieur étant constitué d'un corps de cathéter extérieur (3) comprenant au moins une couche intérieure (34), une couche extérieure (35) et une couche de renforcement (36) intercalée entre elles, un embout mou et souple (4) fixé à une extrémité distale dudit corps de cathéter extérieur, et un moyeu de cathéter extérieur (5) fixé à une extrémité proximale dudit corps de cathéter extérieur ;
ledit cathéter intérieur (6) étant constitué d'une partie proximale dure, d'une partie distale plus molle que ladite partie proximale dure, et d'un moyeu de cathéter intérieur (9) formé à une extrémité proximale de la partie proximale, et
dans lequel ledit moyeu de cathéter extérieur (5) et ledit moyeu de cathéter intérieur (9) sont adaptés pour pouvoir être fixés l'un à l'autre de telle manière qu'ils ne tournent pas et ne se déplacent pas l'un par rapport à l'autre, quand ledit moyeu de cathéter extérieur et ledit moyeu de cathéter intérieur se mettent mutuellement en prise, au moins une partie dudit cathéter intérieur fait saillie depuis une extrémité distale dudit cathéter extérieur, **caractérisé en ce que** la distance entre l'extrémité distale dudit cathéter extérieur et une extrémité distale dudit cathéter intérieur ne dépasse pas 10 mm.

2. Ensemble de cathéter selon la revendication 1, dans lequel le cathéter intérieur comprend un deuxième embout mou (8) possédant une flexibilité à l'extrémité distale.

3. Ensemble de cathéter selon la revendication 2, dans lequel le deuxième embout mou présente une plus grosse épaisseur de paroi que l'embout mou du cathéter extérieur.

4. Ensemble de cathéter selon l'une quelconque des revendications 1 à 3, dans lequel la partie distale du corps de cathéter extérieur comprend une partie courbée prédéterminée.

5. Ensemble de cathéter selon la revendication 4, dans lequel le moyeu de cathéter extérieur est en prise avec le moyeu de cathéter intérieur de telle manière que la limite entre l'extrémité proximale dure et l'extrémité distale souple du cathéter intérieur est située du côté proximal par rapport à la partie courbée du corps de cathéter extérieur.

6. Ensemble de cathéter selon l'une quelconque des revendications 1 à 5, dans lequel le cathéter intérieur a une structure à une seule couche faite d'une résine ne contenant pas de matériau de renforcement sur toute sa longueur.

7. Ensemble de cathéter selon l'une quelconque des revendications 1 à 6, dans lequel le cathéter extérieur a une taille telle que le rapport du diamètre extérieur sur le diamètre intérieur est supérieur ou égal à 0,89 et inférieur ou égal à 0,95.

8. Ensemble de cathéter selon l'une quelconque des revendications 1 à 7, dans lequel le cathéter extérieur a une épaisseur de paroi inférieure à celle du cathéter intérieur.
